# EUROPEAN PATENT SPECIFICATION

(11) **EP 0 960 190 B1**
(45) Date of publication and mention of the grant of the patent: **18.10.2006**
(21) Application number: 97932532.1
(22) Date of filing: 08.07.1997
(51) Int. Cl.: C12N 5/00, C12N 15/00, C12N 15/01, C12N 15/05, A01H 1/00, A01H 1/04, A01H 1/06, A01H 3/00, A01H 4/00, A01H 5/00

(54) **IMIDAZOLINONE HERBICIDE RESISTANT SUGAR BEET PLANTS**
IMIDAZOLIN HERBIZID RESISTENTE ZUCKERRÜBEN
PLANTE DE BETTERAVE A SUCRE RESISTANT AUX HERBICIDES A BASE D'IMIDAZOLINONE

(30) Priority: 17.07.1996 US 683533; 06.12.1996 US 761197
(43) Date of publication of application: 01.12.1999
(73) Proprietor: Michigan State University, East Lansing, MI 48824-1046 (US)
(72) Inventor: PENNER, Donald, Williamston, MI 48895 (US); WRIGHT, Terry, R., Lansing, MI 48910 (US)
(74) Representative: Grünecker, Kinkeldey, Stockmair & Schwanhäusser Anwaltssozietät
(86) International application number: PCT/US1997/011834
(87) International publication number: WO 1998/002527

(56) References cited:
- EP-A- 0 154 204
- US-A- 5 378 824
- STOUGAARD P ET AL: "HERBICIDE RESISTANT MUTANTS OF SUGAR BEET (BETA VULGARIS)" JOURNAL OF CELLULAR BIOCHEMISTRY. SUPPLEMENT, A.R. LISS, NEW YORK, NY, US, no. 14, PART E, 1990, page 310 XP001028988 ISSN: 0733-1959
- HATTORI J ET AL: "MULTIPLE RESISTANCE TO SULFONYLUREAS AND IMIDAZOLINONES CONFERRED BY AN ACETOHYDROXYACID SYNTHASE GENE WITH SEPARATE MUTATIONS FOR SELECTIVE RESISTANCE" MOLECULAR AND GENERAL GENETICS, SPRINGER VERLAG, BERLIN, DE, vol. 232, 1992, pages 167-173, XP002056388 ISSN: 0026-8925
- BERNASCONI P ET AL: "A naturally occurring point mutation confers broad range tolerance to herbicides that target acetolactate synthase" JOURNAL OF BIOLOGICAL CHEMISTRY, AMERICAN SOCIETY OF BIOLOGICAL CHEMISTS, BALTIMORE, MD, US, vol. 270, no. 29, 21 July 1995 (1995-07-21), pages 17381-17385, XP002180518 ISSN: 0021-9258
- WEED SCIENCE, 1992, Vol. 40, HART et al., "Chlorsulfuron-Resistant Sugarbeet: Cross-Resistance and Physiological Basis of Resistance", pages 378-383.
- CROP SCIENCE, 1992, Vol. 32, SAUNDERS et al., "Monogenic Dominant Sulfonylurea Resistance in Sugarbeet from Somatic cell Selection", pages 1357-1360.
- THEOR. APPL. GENET., 1991, Vol. 83, NEWHOUSE et al., "Mutations in Corn (Zea Mays) Conferring Resistance to Imidazolinone Herbicides", pages 65-70

### APPENDIX I

### SEQUENCE LISTING

(1) GENERAL INFORMATION:
   (i) APPLICANT: Donald Penner, Terry R. Wright
   (ii) TITLE OF INVENTION: Imidazolinone Herbicide Resistant Sugar Beet
   (iii) NUMBER OF SEQUENCES: 4
   (iv) CORRESPONDENCE ADDRESS:
      (A) ADDRESSEE: Ian C. McLeod
      (B) STREET: 2190 Commons Parkway
      (C) CITY: Okemos
      (D) STATE: Michigan
      (E) COUNTRY: USA
      (F) ZIP: 48864
   (v) COMPUTER READABLE FORM:
      (A) MEDIUM TYPE: Diskette, 5.25 inch, 360 Kb storage
      (B) COMPUTER: IBM Compatible
      (C) OPERATING SYSTEM: MS-DOS
      (D) SOFTWARE: Wordperfect 5.1
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: 08/683,533
      (B) FILING DATE: July 17, 1996
   (viii) ATTORNEY/AGENT INFORMATION:
      (A) NAME: Ian C. McLeod
      (B) REGISTRATION NUMBER: 20,931
      (C) REFERENCE/DOCKET NUMBER: MSU 4.1-344
   (ix) TELECOMMUNICATION INFORMATION:
      (A) TELEPHONE: (517) 347-4100
      (B) TELEFAX: (517) 347-4103
      (C) TELEX: None
(2) INFORMATION FOR SEQ ID NO:1:
   (i) SEQUENCE CHARACTERISTICS:
      (A) LENGTH: 5
      (B) TYPE: Amino acid
      (C) STRANDEDNESS: Single
      (D) TOPOLOGY: Linear
   (ii) MOLECULE TYPE:
      (A) Description: peptide
   (iii) HYPOTHETICAL: No
   (iv) ANTI-SENSE: No
   (vi) ORIGINAL SOURCE:
      (A) ORGANISM: sugarbeet
      (B) STRAIN: wt
      (C) INDIVIDUAL ISOLATE:
      (G) CELL TYPE: N/A
   (ix) FEATURE:
      (A) NAME/KEY:
      (B) LOCATION:
      (C) IDENTIFICATION METHOD: Sequencing
      (D) OTHER INFORMATION:
   (xi) SEQUENCE DESCRIPTION: SEQ ID NO:1:
   Ala Pro Ala Trp Ser
                     5
(3) INFORMATION FOR SEQ ID NO:2:
   (i) SEQUENCE CHARACTERISTICS:
      (A) LENGTH: 5
      (B) TYPE: amino acid
      (C) STRANDEDNESS: Single
      (D) TOPOLOGY: Linear
   (ii) MOLECULE TYPE:
      (A) Description: peptide
   (iii) HYPOTHETICAL: No
   (iv) ANTI-SENSE: No
   (vi) ORIGINAL SOURCE:
      (A) ORGANISM: sugarbeet
      (B) STRAIN: Sir-13
      (C) INDIVIDUAL ISOLATE: N/A
      (G) CELL TYPE: N/A
   (ix) FEATURE:
      (A) NAME/KEY:
      (B) LOCATION:
      (C) IDENTIFICATION METHOD: Sequencing
      (D) OTHER INFORMATION:
   (xi) SEQUENCE DESCRIPTION: SEQ ID NO:2:
   Thr Pro Ala Trp Ser
                     5
(4) INFORMATION FOR SEQ ID NO:3:
   (i) SEQUENCE CHARACTERISTICS:
      (A) LENGTH: 5
      (B) TYPE: amino acids
      (C) STRANDEDNESS: Single
      (D) TOPOLOGY: Linear
   (ii) MOLECULE TYPE:
      (A) Description: peptide
   (iii) HYPOTHETICAL: No
   (iv) ANTI-SENSE: No
   (vi) ORIGINAL SOURCE:
      (A) ORGANISM: sugarbeet
      (B) STRAIN: Sur
      (C) INDIVIDUAL ISOLATE: N/A
      (G) CELL TYPE: N/A
   (ix) FEATURE:
      (A) NAME/KEY:
      (B) LOCATION:
      (C) IDENTIFICATION METHOD: Sequencing
      (D) OTHER INFORMATION:
   (xi) SEQUENCE DESCRIPTION: SEQ ID NO:3:
   Ala Ser Ala Trp Ser
                   5
(5) INFORMATION FOR SEQ ID NO:4:
   (i) SEQUENCE CHARACTERISTICS:
      (A) LENGTH: 5
      (B) TYPE: amino acids
      (C) STRANDEDNESS: Single
      (D) TOPOLOGY: Linear
   (ii) MOLECULE TYPE:
      (A) Description: peptide
   (iii) HYPOTHETICAL: No
   (iv) ANTI-SENSE: No
   (vi) ORIGINAL SOURCE:
      (A) ORGANISM: sugarbeet
      (B) STRAIN: 9R30B
      (C) INDIVIDUAL ISOLATE: N/A
      (G) CELL TYPE: N/A
   (ix) FEATURE:
      (A) NAME/KEY:
      (B) LOCATION:
      (C) IDENTIFICATION METHOD: Sequencing
      (D) OTHER INFORMATION:
   (xi) SEQUENCE DESCRIPTION: SEQ ID NO:4:
   Thr Ser Ala Trp Ser
                   5

## Description

### BACKGROUND OF THE INVENTION

### (1) Summary of the Invention

The present invention relates to a method for producing sugar beet plants (Beta vulgaris L.) which are resistant to both imidazolinone and sulfonylurea herbicides used for weed control. In particular, the present invention relates to sugar beet plants derived from a susceptible sugar beet by mutation of a gene encoding acetolactate synthase (ALS) also known as acetohydroxyacid synthase (AHAS) using the herbicides sequentially with cells in a culture medium.

### (2) Description of Related Art

The prior art has described the genetic alteration of the acetolactate synthase gene by recombinant means as shown by U.S. Patent Nos. 5,013,659; 5,141,870 and 5,378,824. This type of modification in sugar beet plants is shown by Example IV of the '824 patent. The results were less than satisfactory in producing plants which breed true for the herbicide resistance.

Saunders et al. in Crop Science 32:1357-1360 (1992) also describe the production of the sugar beet plant (CR1-B) which is resistant to sulfonylureas from the susceptible self-fertile clone (REL-1) by selection for mutant cells in a culture medium containing the herbicide. Various resistant plants were produced and cross-bred. Hart et al. (Weed Science 40378-383 (1992) and 41:317-324 (1993)) further characterized the resistant line and determined the resistance was due to altered ALS activity and showed no cross resistance to other ALS-inhibiting herbicides, and was coded for by a single, semidominant gene.

There are no publications describing imidazolinone resistance obtained by modifying ALS in sugar beet plants. There is no description in the literature of plants which are resistant to both imidazolinone and sulfonylurea herbicides. Various corn lines with imidazolinone resistance have been developed as described by Newhouse et al. Theoretical and Applied Genetics 83:65-70 (1991).

A commercial route to crop protection is the use of clinical "safeners" such as described in European Patent No. 375,875. This method introduces another chemical into the soil. The preferred method is to develop sugar beet plants which are resistant to the imidazolinone and sulfonylurea herbicides.

### OBJECTS

It is therefore an object of the present invention to provide a method for imparting resistance to both imidazolinone and sulfonylurea herbicides in sugar beet plants by mutation of a acetolactate synthase gene encoding for this resistance. Further, it is an object of the present invention to provide sugar beet plants which are resistant to these herbicides. These and other objects will become increasingly apparent by reference to the following description and the drawings.

### BRIEF DESCRIPTION OF DRAWINGS

Figure 1 is a schematic view of the method of the present invention for producing imidazolinone and sulfonylurea resistant sugar beet plants 93R30A or B. R = Resistant; IM = imidazolinone; S = sensitive; SU = sulfonylurea; Sur = ALS allele dominant for SU-R, TP-R and IM-S; *SB* = ALS allele dominant for SU-R, TP-R, and IM-R; TP = triazolopyrimidine. 93R30B is SU-R and IM-R and TP-R. 93R30 (not 93R30B) is homozygous for *Sur* allele (SU-R, IM-S). This was the starting material to obtain the imadazolinone resistance.

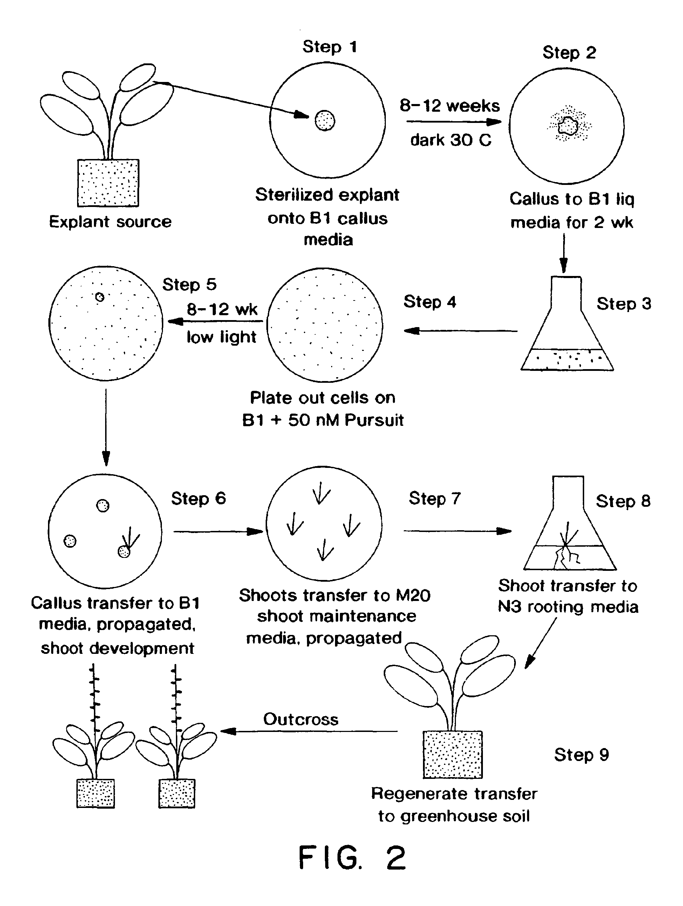

Figure 2 is a flow diagram showing the detailed steps in producing a mutant sugar beet plant.
Figure 3 is a graph showing an ALS assay for 93R30B resistance to imazethapyr.
Figure 4 is a chart showing amino acid substitutions observed for wildtype (*wt*) three mutant sugarbeets (*Sir-13, Sur,* and *SB*) and corresponding herbicide resistance. Only amino acid residues reported in the literature which account for herbicide resistance in plants are included. One letter amino acid codes are as follows: A=alanine, P=proline, W=tryptophan, s-serine, and T=threonine). Boxed letters indicated changes from the wildtype sequence.
Figures 5A to 5D are photographs showing whole plant sugarbeet response to imazethapyr applied postemergence.
Figures 6A to 6D are photographs showing whole plant sugarbeet response to AC 299,263 applied postemergence.
Figure 7 is a photograph showing ALS enzyme response to imazethapyr *in vitro.*
Figure 8 is a photograph showing ALS enzyme response to fumetsulam *in vitro*.
Figures 9A to 9D are photographs showing whole plant sugarbeet response to chlorosulfuron applied postemergence.
Figure 10 is a photograph showing ALS enzyme response to chlorsulfuron *in vitro.*

### DESCRIPTION OF PREFERRED EMBODIMENTS

A sugar beet plant material consisting of mutated cells with a mutated acetolactate synthase gene encoding the synthase wherein nucleotides are modified from cytosine to thymine at position 562 and guanine to adenine at position 337, wherein the mutated cells have a resistance to both imidazolinone and sulfonylurea herbicides and wherein the resistance is transmitted by conventional cross-breeding of plants produced from the cells and the cells are regenerable to a plant.

Further, the present invention relates to a method of producing a herbicide resistance in a sugar beet plant which comprises:
(a) exposing first cells of the sugar beet with a mutated acetolactate synthase gene encoding the synthase, wherein nucleotides are modified from cytosine to thymine at position 562, to an imidazolinone herbicide in a culture medium, wherein the first cells have a homozygous trait for resistance sulfonylurea herbicide; and
(b) selecting for second cells having a resistance to both herbicides, wherein the resistance can be transmitted by cross-breeding of a plant containing the second cells.

Further, the present invention relates to a method for imparting a herbicide resistance in a sugar beet which comprises:
(a) exposing first cells of sugar beet plant to an imidazolinone herbicide in a culture medium, wherein the first cells have a homozygous resistance sulfonylurea herbicide in a culture medium;
(b) selecting for second cells having a resistance to both herbicides;
(c) growing a first plant from the second cells, wherein the plant has the resistance to the herbicides with a mutated acetolactate synthase gene encoding the synthase, wherein nucleotides are modified from cytosine to thymine at position 562 and guanine to adenine at position 337; and
(d) cross-breeding the first plant with a second plant to produce a crossed plant which has the resistance to the herbicides; and finally to a method for imparting herbicide resistance to other beets which comprises:
   (a) exposing first cells of sugar beet plant to an imidazolinone herbicide in a culture medium, wherein the first cells have a homozygous resistance sulfonylurea herbicide in a culture medium;
   (b) selecting for second cells having a resistance to both herbicides;
   (c) growing a first plant from the second cells, wherein the plant has the resistance to the herbicides with a mutated acetolactate synthase gene encoding the synthase, wherein nucleotides are modified from cytosine to thymine at position 562 and guanine to adenine at position 337; and
   (d) cross-breeding the first plant with a second plant to produce a crossed plant which has the resistance to the herbicides.

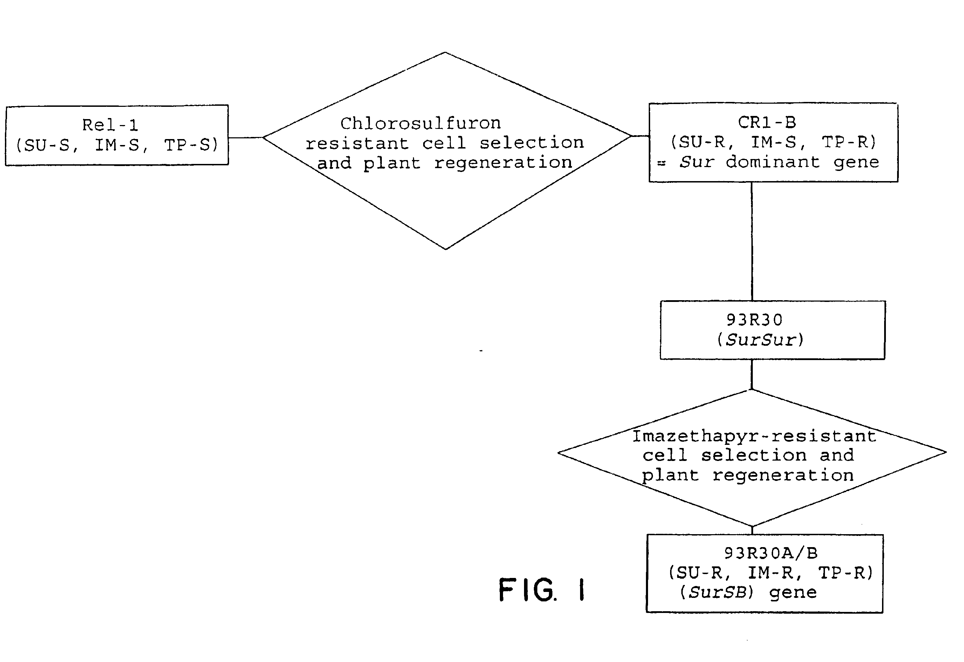

The sugar beet plant REL-1 (Regenerating, East Lansing-1) is available from Dr. Joseph Saunders, Research Geneticist, U.S. Department of Agriculture, East Lansing, Michigan and was released in 1987. It is available without cost. REL-1 is sensitive (S) to imidazolinone (IM-S), sulfonylurea (SU-S), and triazolopyrimidine sulfonanilide (TP-S) herbicides as indicated in Figure 1.

Using callus and suspension cultures a line which was heterozygous for imidazolinone and sulfonylurea resistance (IM-R and SU-R) was developed. This cell line (seed) has been deposited with the American Type Culture Collection as ATCC 97535 on May 6, 1996 under the Budapest Treaty and is referred to herein as 93R30B. The cell line is available upon request by name and number. By breeding with elite sugar beet plant lines, in particular *Beta vulgaris L.,* especially commercially useful resistant sugar beet plants can be produced.

Sugar beet plants resistant to the imidazolinone herbicides, specifically imazethapyr, and sulfonylurea herbicides (chlorsulfuron) solve the soil residue problem from the use of that herbicide in sugar beet growing areas. Currently, there is a 40-month waiting period between the use of that herbicides and the future use of that field for growing sugar beet plants. Secondly, a high level of imidazolinone and sulfonylurea resistance allows use of the imidazolinone herbicides for weed control in sugar beets.

The following Examples show isolation and breeding of a novel sugar beet plant 93R30B as illustrated in Figure 1.

### EXAMPLE 1

### 1. Selection of imidazolinone and sulfonylurea resistant sugar beet variant 93R30B.

### Description of Materials

- Rel-1: (Regenerating East Lansing-1) is a highly regenerable male fertile sugar beet line used for initial cell selection for herbicide-resistant mutant to the sulfonylureas and is used as a sensitive control for most experiments.
- CR1-B: This sugar beet isolate was developed by plating Rel-1 cells on chlorsulfuron-containing media. This variant is sulfonylurea resistant with modest resistance to the triazolopyrimidine herbicide, flumetsulam, but no imidazolinone resistance. This variant has been described by Saunders et al. (Crop Sci. 32:1357-1360) and Hart et al. (Weed Sci. 40:378-383 and 41:317-324). The SU resistance gene has been labeled Sur.
- 93R30B: This isolate was developed by plating cells of a highly regenerable, Sur homozygous sugar beet plant (descendant of CR1-B) on media containing imazethapyr.
- EL-49: East Lansing 49, released by the USDA in 1993. Sugar beet line homozygous for the Sur gene, used for a sulfonylurea-resistant, imazethapyr- sensitive control.

a. Plants were selected as source materials for somaclonal selection based on their ability to generate callus under high cytokinin conditions (in B1 media) and regenerate shoots from callus.
   Protocol for B1 media:
   30 g L⁻¹ sucrose
   100 mg L⁻¹ myo-inositol
   1.65 g L⁻¹ NH₄NO₃
   1.90 g L⁻¹ KNO₃
   0.44 g L⁻¹ CaCl₂.2H₂O
   0.37 g L⁻¹ MgSO₄.7H₂O
   0.17 g L⁻¹ KH₂PO₄
   6.2 mg L⁻¹ H₃BO₃
   16.8 mg L⁻¹ MnSO₄.H₂O
   10.6 mg L⁻¹ ZnSO₄.7H₂O
   0.88 mg L⁻¹KI
   0.25 mg L⁻¹ Na₂MoO₄.2H₂O
   0.025 mg L⁻¹ CuSO₄.5H₂O
   0.025 mg L⁻¹ CoCl₂.6H₂O
   37.3 mg L⁻¹ Na₂EDTA
   27.8 mg L⁻¹ FeSO₄.7H₂O
   1 mg L⁻¹ thiamine
   0.5 mg L⁻¹ pyridoxine
   0.5 mg L⁻¹ nicotinic acid
   1 mgL⁻¹ benzylaminopurine
   pH 5.95
   B1 solid media was autoclaved with 9 g L⁻¹ plant culture agar and modified with filter-sterilized herbicide stock solutions as needed for selection scheme. Agar media was poured into 15 x 100 mm disposable plastic petri dishes.
   B₁ liquid media was added in 40 ml aliquots to 125 ml Erlenmeyer flasks and autoclaved for use in liquid suspension cultures.
   For selection of the imidazolinone resistant variant, 93R30B, the source material was 93R30 (a *Sur* homozygotic descended from CR1-B).
   In particular:
   1. Cells from Rel-1 (SU-S, IM-S, TP-S) were placed into culture where cells were selected for their ability to grow on chlorsulfuron and a plant was regenerated, CR1-B (described by Saunders et al, Crop Sci, 32:1357-1360 (1992)).
   2. CR1-B was crossed with 293 (a more typical beet), the F1 selfed and homozygous plants for SU-R, IM-S, TP-R were identified. A plant known as EL-49 meeting this description was released in 1993 by Saunders (the gene was called Sur and this was homozygous for it).
   3. At a later date, descendants of CR1-B homozygous for Sur were selected for their ability to regenerate plants from culture. They then served as source material (e.g., 93R30) for imazethapyr selection in culture.
   4. From the above selection, the isolates known as 93R30A and 93R30B were derived and plants regenerated. These had the characteristics of CR1-B plus IM-R. The gene responsible for IM-R appears to be an altered form of *Sur* and has been given the identity of *SB.*

   Leaf disk explants were prepared from rapidly expanding leaves of the source plant (93R30). The leaves were surface sterilized (step 1) by two successive 20-minute washes with 15% commercial bleach plus 0.025% Triton X-100 (T-9280, Sigma Chem. Co., St. Louis, MO). Leaves were rinsed twice with sterile deionized water. Leaf disks were cut using a flame-sterilized #3 cork borer.
   Leaf disks were placed aseptically onto solid B1 media (step 2). Disks were incubated in the dark at 30°C for 4-8 weeks until friable, white callus tissue had proliferated from the leaf disk. Callus tissue were separated mechanically with forceps and subsequently transferred to 125 ml Erlenmeyer flasks (step 3) containing 40 ml liquid B1 media. Flasks were placed onto a gyratory shaker at 50 Hz under low intensity fluorescent lighting (30 µE/m² s). Liquid cultures were subcultured with fresh B1 media after 1 week.
   Two weeks after liquid culture initiation, cell clumps were separated using a cell dissociation sieve (Sigma CD-1) with a 60 mesh screen. Approximately two-thirds volume of the liquid media was removed following cell sedimentation. In step 4, cells were resuspended in remaining media and 1 ml aliquots were spread evenly over solid B1 media supplemented with 50 nM imazethapyr (PURSUIT, American Cyanamid, Wayne, NJ) herbicide. Plates were wrapped and incubated in dim fluorescent light (5-10 µE/m² s) for 8-12 weeks.
c. At 50 nM imazethapyr concentration, all sensitive cells died. Cell clumps that survived and grew at this concentration are identified as possible resistant variants. In step 5, these cell clumps (1-3 mm in diameter) were transferred to fresh B1 solid media without herbicide and allowed to grow under low light (10-20 µE/m² s). Each putatively resistant variant was individually identified and maintained separately.
d. In step 6, white, friable callus was subdivided every 4-6 weeks to fresh B1 solid media until an individual shoot(s) grew from the callus. These shoots were transferred to shoot maintenance media (M20) as follows:
   Protocol for M20 media:
   30 g L⁻¹ sucrose
   100 mg L⁻¹ myo-inositol
   1.65 g L⁻¹ NH₄NO₃
   1.90 g L⁻¹ KNO₃
   0.44 g L⁻¹ CaCl₂.2H₂O
   0.37 g L⁻¹ MgSO₄.7H₂O
   0.17 g L⁻¹ KH₂PO₄
   6.2 mg L⁻¹ H₃BO₃
   16.8 mg L⁻¹ MnSO₄.H₂O
   10.6 mg L⁻¹ ZnSO₄.7H₂O
   0.88 mg L⁻¹ KI
   0.25 mg L⁻¹ Na₂MoO₄.2 H₂O
   0.025 mg L⁻¹ CuSO₄.5 H₂O
   0.025 mg L⁻¹ CoCl₂.6H₂O
   37.3 mg L⁻¹ Na₂EDTA
   27.8 mg L⁻¹ FeSO₄.7 H₂O
   1 mg L⁻¹ thiamine
   0.5 mg L⁻¹ pyridoxine
   0.5 mg L⁻¹ nicotinic acid
   0.25 mg L⁻¹ benzylaminopurine
   pH 5.95
M20 solid media was autoclaved with 9 g L⁻¹ plant culture agar and modified with filter-sterilized herbicide stock solutions as needed for selection scheme. Agar was poured into 20 x 100 mm disposable plastic petri dishes.

Shoot cultures were maintained under 10-20 µE/m² s fluorescent lighting and propagated by dissecting shoot cultures with a scalpel blade. Candidate shoot cultures were propagated and assessed for level of resistance as described hereinafter.

### 2. Level of imidazolinone, sulfonylurea and triazolopyrimidine sulfonanilide resistance in shoot culture.

In step 7, shoot cultures were assessed for herbicide resistance by placing three small, consistently sized shoot cuttings on each plate of M20 media supplemented with logarithmically increasing concentrations of imidazolinone herbicide, sulfonylurea or triazolopyrimidine sulfonanilide ranging from 1 nM to 0.1 mM. 93R30 culture responses were compared to that of shoot cultures of a sensitive control (Rel-1) and a chlorsulfuron resistant, imidazolinone sensitive control (EL-49). Shoot cultures were maintained at 20 µE/m² s light intensity at 25°C for 3 weeks. Shoot culture was determined by rating shoot injury and fresh weight 3 weeks after transfer to selective media. Magnitude of herbicide resistance was determined by the ratio of resistant to sensitive I₅₀ values (the herbicide concentration causing 50 percent injury).

The cross resistance characteristics of each variant were determined in the manner above by substituting the herbicide used in the media. In shoot culture 93R30B showed a 3600 fold level of resistance to imazethapyr, a 10,000 fold resistance to chlorsulfuron and showed a 60 fold cross resistance to the triazolopyrimidine sulfonanilide herbicide, flumetsulam as shown in Table 1. For comparison EL-49 showed only a 3 fold level of resistance to imazethapyr.

**TABLE 1**

| Magnitude of shoot culture resistance and cross-resistance: | | | | | | |
|---|---|---|---|---|---|---|
| Herbicide | Herbicide family⁶ | Rel-1 | 93R30B | | EL-49 | |
| | | I₅₀⁵(µM) | I₅₀ (µM) | R/S⁴ | I₅₀(µM) | R/S |
| Imazethapyr | IM | 0.018 | 64 | 3600X | 0.053 | 3X |
| Imazamethabenzmethyl | IM | 0.80 | > 100 | > 125X | 20 | 25X |
| Imazaquin | IM | 0.040 | 16 | 400X | 0.040 | 1X |
| AC299,263¹ | IM | 0.030 | 40 | 1400X | 0.070 | 3X |
| Chlorsulfuron² | SU | 0.0025 | 25 | 10000X | 25 | 10000X |
| Flumetsulam³ | TP | 0.028 | 1.7 | 60X | 1.1 | 40X |

| | | | | | | |
|---|---|---|---|---|---|---|
| ¹ - Imadazolinone RAPTOR (American Cyanamid, Wayne, NJ) ² - Sulfonylurea - GLEAN (Dupont, Wilmington, DE) ³ - Triazolopyrimidine sulfonanilide (BROADSTRIKE, Dow Elanco, Indianapolis, IN) ⁴ - R/S = ratio of resistant to sensitive control I₅₀ values for the specific herbicide being tested. ⁵ - I₅₀ is the concentration of herbicide required to cause 50% injury to the shoot culture. ⁶ - Herbicide family classifications: IM, imidazolinone; SU, sulfonylurea; and TP, triazolopyrimidine sulfonanilide. | | | | | | |

### 3. Plant regeneration and breeding.

In step 8, shoot cultures of 93R30B were regenerated to whole plants in a similar manner. Two weeks following previous shoot subculture, 93R30B shoots were transferred to 125 ml Erlenmeyer flasks containing 40 ml N3 rooting media.
Protocol for N3 media:
30 g L⁻¹ sucrose
100 mg L⁻¹ myo-inositol
1.65 g L⁻¹ NH₄NO₃
1.90 g L⁻¹ KNO₃
0.44 g L⁻¹ CaCl₂.2H₂O
0.37 g L⁻¹ MgSO₄.7H₂O
0.17 g L⁻¹ KH₂PO₄
6.2 mg L⁻¹ H₃BO₃
16.8 mg L⁻¹ MnSO₄.H₂O
10.6 mg L⁻¹ ZnSO₄.7H₂O
0.88 mg L⁻¹ KI
0.25 mg L⁻¹ Na₂MoO₄.2H₂O
0.025 mg L⁻¹ CuSO₄.5H₂O
0.025 mg L⁻¹ CoCl₂.6H₂O
37.3 mg L⁻¹ Na₂EDTA
27.8 mg L⁻¹ FeSO₄.7H₂O
1 mg L⁻¹ thiamine
0.5 mg L⁻¹ pyridoxine
0.5 mg L⁻¹ nicotinic acid
3 mg L⁻¹ naphthalene acetic acid
pH 5.95
N3 media added in 40 ml aliquots to 125 ml Erlenmeyer flasks, 9 g L⁻¹ agar (0.45 g) added to each flask, and autoclaved for use in rooting of shoot cultures. Cultures were then transferred to 24 hour per day light under medium light intensity (40-60 µE/m² s) at 25°C. Roots generally formed 6-8 weeks later.

In step 9, rooted shoots (R₀ generation) were then transferred to Baccto (Michigan Peat Co., Houston, TX) potting mix in the greenhouse. In step 10, R₀ plants of 93R30B regenerates were crossed with a smooth-root sugar beet called 293 or with REL-1. F₁ seed from these crosses were self-fertilized to yield F₂ seed. It was presumed that imidazolinone resistance was a dominant or semi-dominant monogenic trait. F₂ generation plants resulting from self fertilization of imidazolinone and sulfonylurea-resistant F₁ plants should be segregating in a 1 homozygous resistant: 2 heterozygous resistant: 1 homozygous sensitive ratio for both imidazolinone and sulfonylurea resistances.

Evidence suggests that imidazolinone resistance is a dominant trait in 93R30B. This conclusion is derived from evidence examining F₁ progeny of crosses between 93R30B and herbicide sensitive, male fertile sugar beet lines 293 or Rel-1 which segregate in a 1:1 fashion for IM-R or IM-S. Likewise, in all F₂ or Backcross (BC₁) plants tested to date, IM-R and SU-R co-segregate. This suggests these two resistances are linked and due to separate alterations to the same ALS allele. Resistance or sensitivity of F₁ plants to imazethapyr and chlorsulfuron has been determined using a non-destructive leaf disk expansion assay.

### Assay Protocol:

This protocol is a non-destructive test designed to screen beets segregating for herbicide resistance early to determine their status as resistant or susceptible without having to spray the beets postemergence in the greenhouse.
1. Newly expanding leaf was excised from plant (at least the 4th true leaf).
2. The leaf was surface sterilized with two 20-minute washes with 15% commercial bleach + 0.025% Triton X-100, then rinsed twice with sterile distilled water.
3. Leaf disk explants were cut with flame-sterilized #3 cork borer.
4. Leaf disks transferred to B1 media containing no herbicide, 100 nM imazethapyr or 100 nM chlorsulfuron. Plates were divided into four sections so four samples an be tested with a single plate. Four to five leaf disks were added to each plate for each sample.
5. Plates were wrapped and incubated at 25°C for 4-7 days under low light (approximately 10 µE/m² s).
6. Resistant samples were identified by their ability to live and expand on the selective media. Sensitive samples will not expand and will turn yellow to brown. Effects were compared to the expansion of leaf disks placed on no-herbicide media to insure accurate determination of the expected level of disk expansion.

### 4. ALS target enzyme response to imazethapyr

Standard procedures were utilized to partially purify ALS from rapidly expanding leaves of greenhouse-grown sugar beet plants (Hart, et al. Weed Science 40:378-383 (1992)). Extracts from heterozygous 93R30B F₁ plants and Rel-1 S₁ seed were assayed for ALS activity in the presence of logarithmically increasing concentrations of imazethapyr. ALS activity was determined from leaf extracts of REL-1 and 93R30B sugarbeet lines. Plants were grown in the greenhouse as described above to the four to six leaf stage. ALS activity from fresh extracts was determined in the presence of imazethapyr and chlorsulfuron at logarithmically increasing concentrations. The methods and procedures used were modified from those outlined by Ray (Plant Physiol. 75:827-831 (1984)) and Shaner, et al. (Plant Physiol. 76:545-546 (1984)). Ten g of sugarbeet leaves were homogenized in 40 ml cold homogenization buffer (0.1 M K₂HPO₄, pH 7.5, 1 mM sodium pyruvate, 0.5 mM MgCl₂, 0.5 mM thiamine pyrophosphate, 10 µM of flavin adenine dinucleotide, 10% by vol glycerol) plus 2.5 g polyvinylpolypyrolidone. The homogenate was filtered through eight layers of cheesecloth and centrifuged at 27,000 g for 20 minutes. The supernatant was removed and brought to 50% saturation with (NH₄)₂SO₄. This solution was kept at 0°C for 1 hour, then centrifuged at 18,000 g for 15 minutes, the pellet redissolved in 1 ml resuspension buffer (0.1 M K₂HPO₄, pH 7.5, 20 mM sodium pyruvate, 0.5 mM MgCl₂) and desalted on a Sephadex G-25 PD-10⁵ column. The enzyme extracts were assayed immediately.

ALS activity is measured by adding 0.2 ml of enzyme preparation (diluted 3:1 with resuspension buffer) to 1.3 ml of reaction buffer (25 mM K₂HPO₄, pH 7.0, 0.625 mM MgCl₂, 25 mM sodium pyruvate, 0.625 mM thiamine pyrophosphate, 1.25 µM flavin adenine dinucleotide) and incubated at 35°C for 1 hour. Reaction mixtures contained a final concentration of 0, 4, 40, 400, 4000, 40000, 400000, or 4000000 nM imazethapyr or 0, 0.9, 9, 90, 900, 9000, 90000 nM chlorsulfuron. The reaction was stopped by adding 50 µl of 6 N H₂SO₄ and incubating at 60°C for 15 minutes. This procedure as described by Westerfield (J. Biol. Chem. 16:495-502 (1945)) also decarboxylates the ALS enzyme product, acetolactate, to form acetoin. A colored acetoin complex was formed by adding 1 ml 2.5% by weight α-naphthol and 0.25% by weight creatine in 2.5 N NaOH and incubating at 60°C for 15 minutes.

Purchased acetoin was used as a standard for the colorimetric reaction. Acetoin concentrations were determined by measuring the absorption of the reaction solution at 530 nm. Experiments with each herbicide were repeated with three replications in each. Protein concentrations of the extracts was determined by the method of Bradford (Anal. Biochem. 72:248-254 (1976)) using bovine serum albumin for the standard curve.

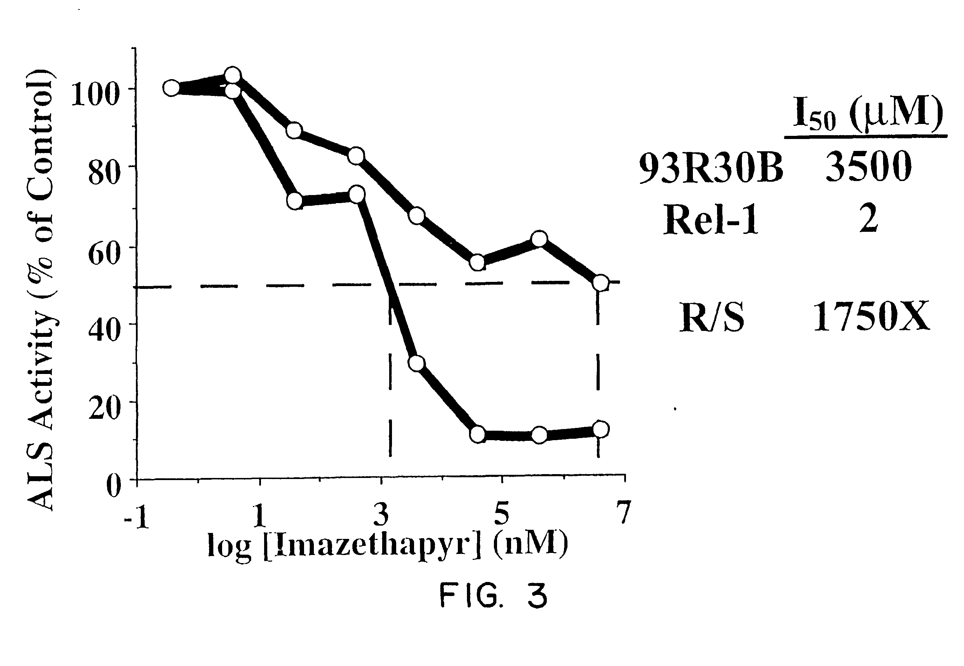

Experiments were conducted twice and treatments replicated three times. Figure 3 shows the response of ALS extracts from 93R30B and the sensitive Rel-1. 93R30B shows a 1750-fold level of resistance to imazethapyr at the enzyme level (as determined by the ratio of I₅₀'s for the resistant and sensitive lines).

### 5. ALS gene copy number

Southern blot analysis was conducted to determine the number of ALS gene copies present in sensitive sugar beet. Genomic DNA was isolated from sensitive F₃ plants descended from the original Sir-13 mutant and analyzed using commonly practiced techniques (Current Protocols in Molecular Biology, J. Wiley and Sons, New York (1991)). Likewise, genomic DNA from a commercial sugar beet variety was analyzed. In both sugar beet lines, a single copy of the ALS gene was detected. This data would indicate that all ALS enzyme activity of a homozygous mutant-type sugar beet would consist of the resistant enzyme form. This also supports the belief that the *SB* change observed in 93R30B isolate is an altered form of the Sur ALS allele. This was confirmed as described below.

### 6. ALS gene mutation

To determine the molecular basis for ALS enzyme resistance, standard techniques were used to sequence two regions of the ALS gene where all previously reported herbicide resistance mutations have occurred (Current Protocols in Molecular Biology, J. Wiley and Sons, New York (1991)). The sugar beet ALS gene had previously been sequenced (U.S. Patent 5,378,824) and Polymerase Chain Reaction (PCR) primers designed to amplify the two regions of the gene responsible for previously reported cases of plant herbicide resistance. Comparison of sequence data from SU-R IM-S TP-R (*Sur*), SU-R IM-R TP-R (*SB*), and sensitive (Rel-1) sugar beets indicated 93R30B's herbicide resistance traits were due to two independent mutations in the ALS gene.

The mutation causing SU-R IM-S TP-R (Sur) is the result of a single nucleotide change from cytosine to thymine at position 562 in the nucleotide sequence which results in a serine for proline substitution at position 188 in the sugar beet ALS amino acid sequence. This site has previously been implicated in sulfonylurea resistance in *Arabidopsis thaliana* (Haughn et al., Molecular and General Genetics 211:266-271 (1988)), tobacco (Lee, et al., EMBO J. 7:1241-1248 (1988)), and in yeast (U.S. Patent 5,378,824). No other bases changes were observed from wild type nucleotide sequence in the two regions of the ALS gene examined.

The mutation causing SU-R IM-R TP-R (*SB*) is the result of two independent mutations in the ALS nucleotide sequence resulting in two different amino acid changes. The first change is identical to the *Sur* mutation described above. This observation was expected since a sugarbeet plant (93R30) served as the starting material for IM-R selection. In addition to this point mutation, an additional single nucleotide change from guanine to adenine at position 337 was observed. This second mutation results in a threonine for alanine substitution at position 113 in the sugar beet ALS amino acid sequence. This site has previously been implicated in imidazolinone resistance in cocklebur (Bernasconi et al., J. Biol. Chem. **270**:17381-17385 (1995)) and for sulfonylurea resistance in yeast (U.S. Patent 5,378,824). No other bases changes were observed from wild type nucleotide sequence in the two regions of the ALS gene examined.

The following information shows herbicide resistant synergism of possible Ala₁₁₃ →Thr and Pro₁₈₈ →Ser mutations.

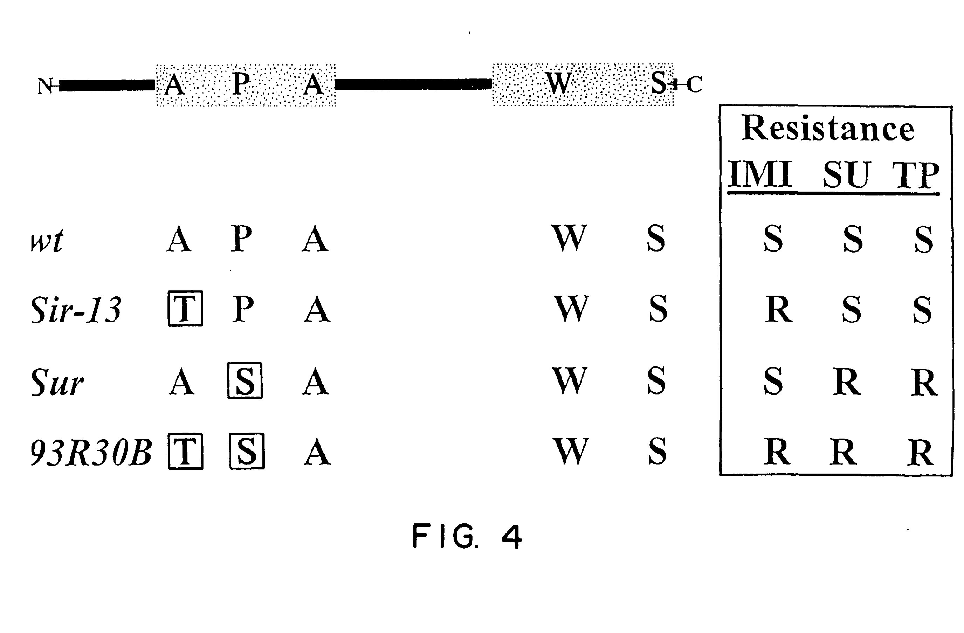

The *SB* sugarbeet ALS gene double mutation provides herbicide resistance to the combination of the chemical families afforded by each of the single mutant sugarbeets individually (i.e., *Sur* and *Sir-13*). Figure 4 displays the basic family cross resistance characteristics of the two single mutants (*Sur* and *Sir-13*) and the double mutant (*SB*). The amino acids depicted in Figure 4 represent the 5 strictly conserved residues reported in the literature to be involved in ALS-inhibiting herbicide resistance in plants. The interaction of the independent mutations within the double mutant enzyme (and corresponding plants) is unique. The combination of the IM-specific resistance seen with the *Sir*-*13* amino acid substitution (Ala₁₁₃ →Thr) and the SU- and TP-resistance observed with the *Sur* substitution (Pro₁₈₈ →Ser) result in a sugarbeet 93R30B (a.k.a. *SB*) resistant to all three classes of herbicide chemistry.

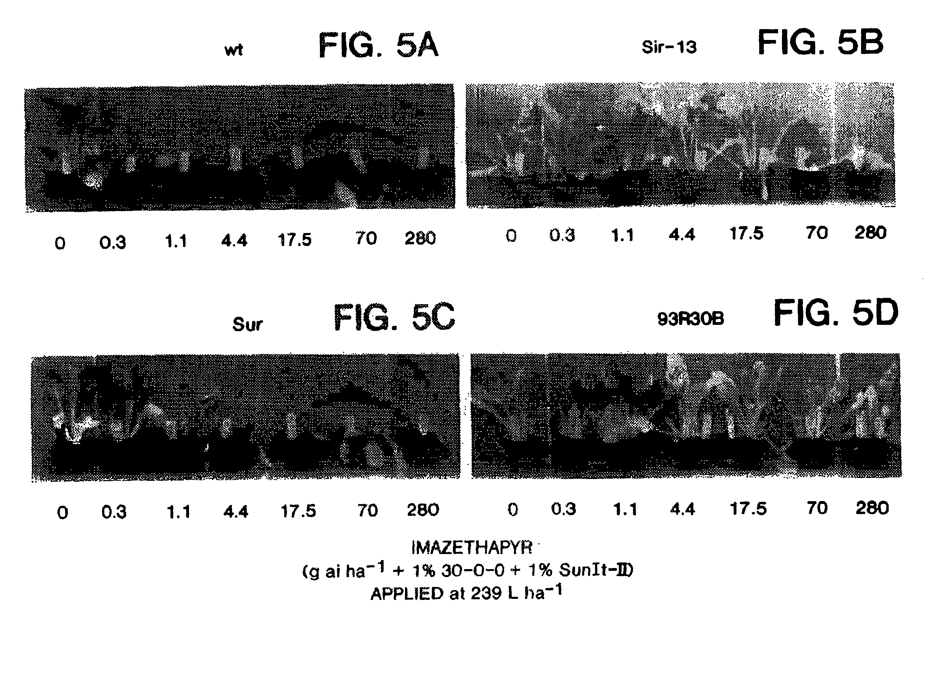

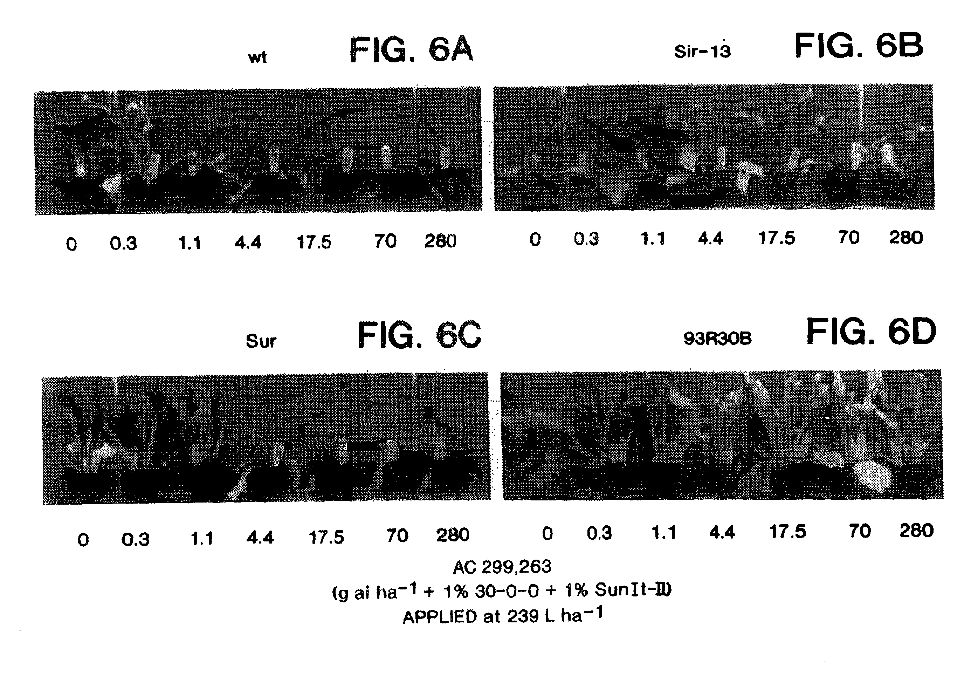

Resistances, however, are not additive. Instead, the mutations are synergistic with respect to resistance to the imidazolinone herbicides imazethapyr and AC 299,263. This phenomenon can be seen visually in Figures 5 and 6 and quantitatively in Table 2.

**TABLE 2**

| **Whole PlantSugarbeet Response to POST ALS-Inhibiting Herbicides** | | | | | |
|---|---|---|---|---|---|
| | | Sugarbeet Line | | | |
| | | Rel-1 | Sir-13 | Sur | 93R30B |
| Herbicide | Class | I₅₀ (g ha⁻¹) | R/S | R / S | R / S |
| Imazethapyr | IMI | 0.5 | 100 X | 3 X | 300 X |
| AC 299,263 | IMI | 1.1 | 130 X | 4 X | >250 X |
| Chlorsulfuron | SU | 0.07 | 1 X | >1000 X | 20 X |
| Sulfometuron | SU | 0.2 | 1 X | 23 X | 4X |

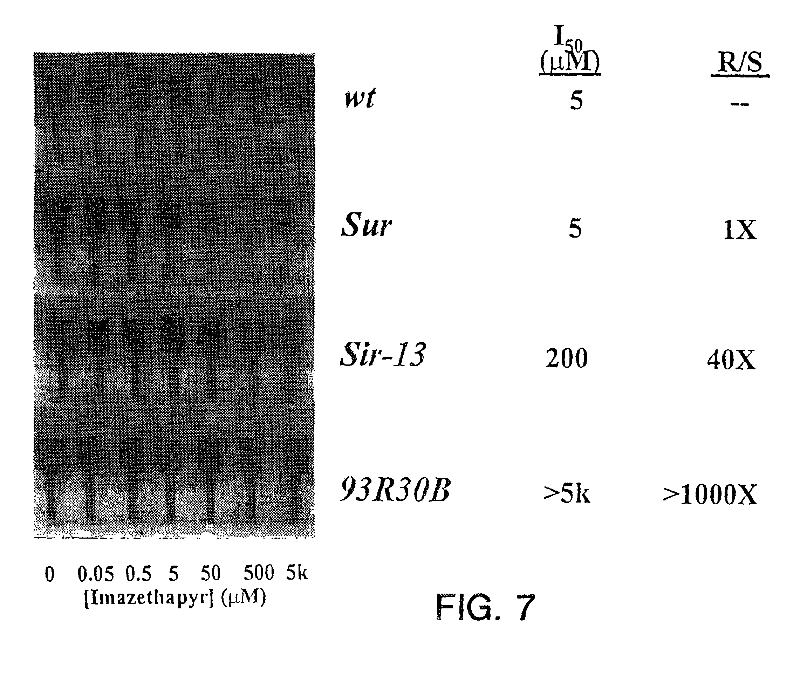

The single mutation sugarbeets, *Sur* and *Sir-13,* exhibit 3 and 100 fold resistance to imazethapyr; whereas, the double mutant, *SB*, is 300 fold resistant to the herbicide when applied postemergence to whole plants. Support for this synergism is provided by observed response of the ALS enzyme from each of the mutants to imazethapyr (Figure 7). Again, a synergistic resistance to the herbicide in the double mutant, *SB* (>1000X), versus the single mutants, Sur (1X) and *Sir-13* (40X), alone.

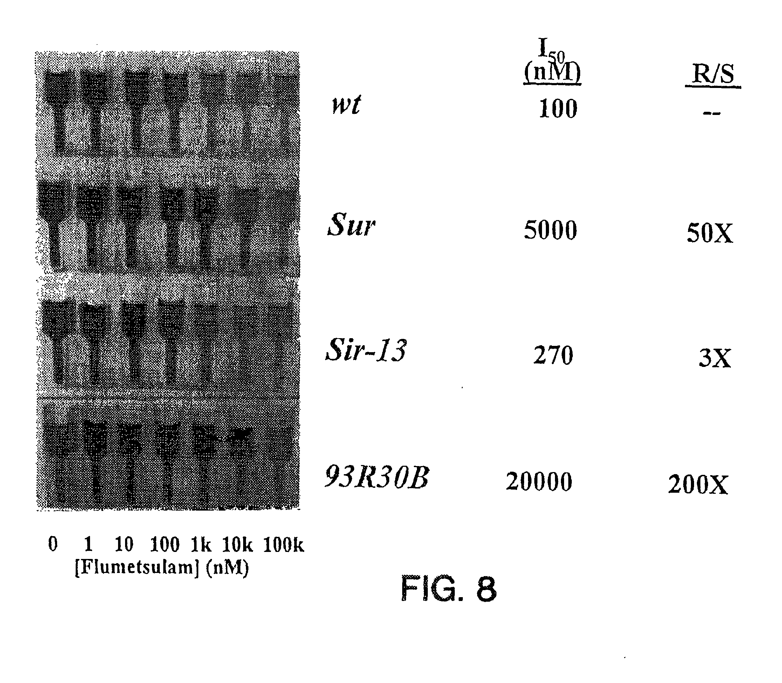

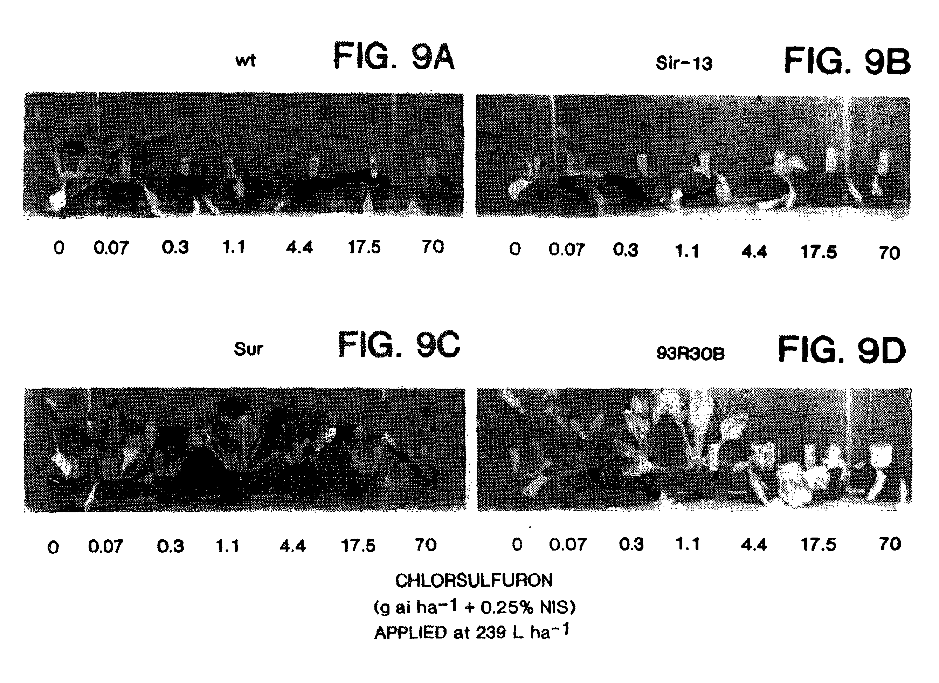

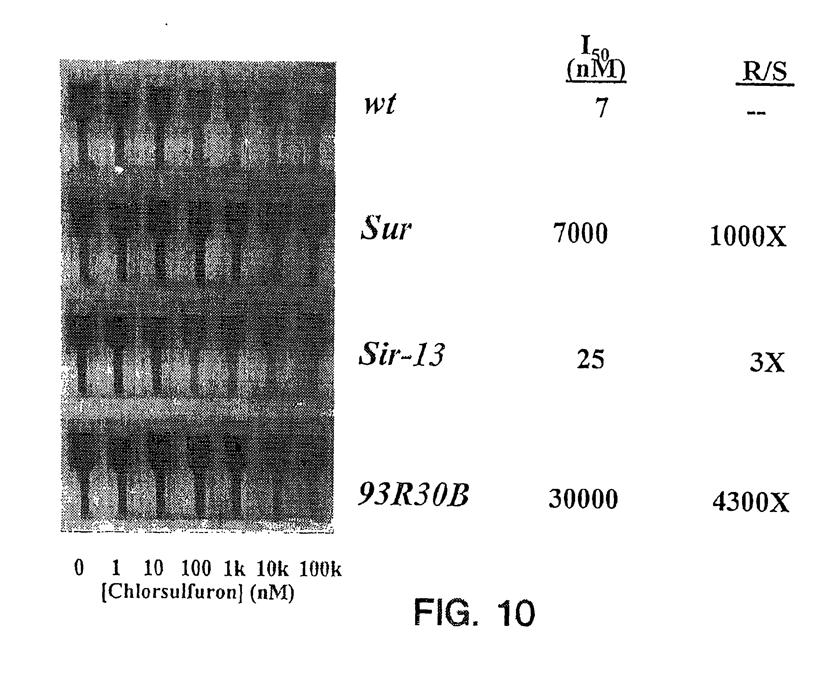

A similar synergism is apparent for the TP class of herbicides. Shoot resistance for the single mutants Sur and *Sir-13* are 40X and 1X, respectively, and 60X for the double mutant, *SB*. ALS enzyme data support this synergism observation with 50X and 3X resistance to flumetsulam for the single mutants Sur and *Sir-13,* respectively, and 200X resistance for the double mutant, *SB* (Figure 8). The second mutation observed in *SB* actually reduces the resistance to chlorsulfuron (a SU class herbicide) observed at the whole-plant level (Figure 9). For chlorsulfuron, whole plant resistance of the *SB* double mutant (20X) is reduced by more than 50 fold by the addition of the *Sir-13*-equivalent mutation (Ala₁₁₃ →Thr) (1X resistance) to the SU-resistance gene, *Sur* (Pro₁₈₈ →Ser) (>1000X resistance) (Table 2). The cause of antagonism between the *Sur* single mutant and the *SB* double mutant as yet is unknown. At the ALS enzyme level, a synergistic resistance is also observed with chlorsulfuron (Figure 10).

It will be appreciated that the selection for imidazolinone resistance can be accomplished first and then the sulfonylurea resistance can be accomplished.

It is intended that the foregoing description be only illustrative of the present invention and that the present invention be limited only by the hereinafter appended claims. Amino acid sequences of Figure 4 are shown in SEQ ID NOS: 1, 2, 3 and 4.

## Claims

1. A sugar beet plant material consisting of mutated cells with a mutated acetolactate synthase gene encoding the synthase wherein nucleotides are modified from cytosine to thymine at position 562 and guanine to adenine at position 337, wherein the mutated cells have a resistance to both imidazolinone and sulfonylurea herbicides and wherein the resistance is transmitted by conventional cross-breeding of plants produced from the cells and the cells are regenerable to a plant.

2. The material of claim 1 which has been derived from sensitive cells of a parent sugar beet plant, designated as CR1-B deposited as ATCC 97961 and having resistance to sulfonylurea herbicides and no resistance to imidazolinone herbicides, by cultivation of the sensitive cells in a culture medium with the imidazolinone herbicide to select for the mutated cells.

3. The plant material of any one of claims 1 or 2 as a seed or propagule of the seed.

4. A method of producing a herbicide resistance in a sugar beet plant which comprises:
(a) exposing first cells of the sugar beet with a mutated acetolactate synthase gene encoding the synthase, wherein nucleotides are modified from cytosine to thymine at position 562, to an imidazolinone herbicide in a culture medium, wherein the first cells have a homozygous trait for resistance sulfonylurea herbicide; and
(b) selecting for second cells having a resistance to both herbicides, wherein the resistance can be transmitted by cross-breeding of a plant containing the second cells.

5. The method of claim 4, wherein the second cells are derived from the first cells of a sugar beet plant designated as 93R30 (SurSur), which is a homozygotic descendant from CR1-B, the latter being deposited as ATCC 97961, by exposing the cells of 93R30 to an imidazolinone herbicide.

6. The method of claim 4 wherein the second cells are in a deposit of a seed designated as ATCC 97535 (93R30B).

7. A method for imparting a herbicide resistance in a sugar beet which comprises:
(a) exposing first cells of sugar beet plant to an imidazolinone herbicide in a culture medium, wherein the first cells have a homozygous resistance sulfonylurea herbicide in a culture medium;
(b) selecting for second cells having a resistance to both herbicides;
(c) growing a first plant from the second cells, wherein the plant has the resistance to the herbicides with a mutated acetolactate synthase gene encoding the synthase, wherein nucleotides are modified from cytosine to thymine at position 562 and guanine to adenine at position 337; and
(d) cross-breeding the first plant with a second plant to produce a crossed plant which has the resistance to the herbicides.

8. A method for imparting herbicide resistance to other beets which comprises:
(a) exposing first cells of sugar beet plant to an imidazolinone herbicide in a culture medium, wherein the first cells have a homozygous resistance sulfonylurea herbicide in a culture medium;
(b) selecting for second cells having a resistance to both herbicides;
(c) growing a first plant from the second cells, wherein the plant has the resistance to the herbicides with a mutated acetolactate synthase gene encoding the synthase, wherein nucleotides are modified from cytosine to thymine at position 562 and guanine to adenine at position 337; and
(d) cross-breeding the first plant with a second plant to produce a crossed plant which has the resistance to the herbicides.

9. The method of claim 7, wherein the first cells in step (a) are derived form a sugar beet designated as 93R30, which is a homozygotic descendant from CR1-B, the latter being deposited as ATCC 97961, by exposing the cells of 93R30 to an imidazolinone herbicide.

10. The method of claim 7 wherein the second cells are in a deposit of a seed designated as ATCC 97535 (93R30B).

11. A method for controlling weeds growing with sugar beet plants which comprises:
(a) planting in a field sugar beet seeds containing cells with a mutated acetolactate synthase gene encoding the synthase wherein nucleotides are modified from cytosine to thymine at position 562 and guanine to adenine at position 337, wherein the mutated cells have a resistance to both imidazolinone and sulfonylurea herbicides and wherein the resistance is transmitted by conventional cross-breeding of plants produced from the cells to produce sugar beet plant; and
(b) using an imidazolinone herbicide on the plant in the field to control weeds.

12. The method of claim 11 wherein the plant is derived from sensitive cells of a parent sugar beet plant, designated as CR1-B deposited as ATCC 97961 and having resistance to sulfonylurea herbicides and no resistance to imidazolinone herbicides, by cultivation of the sensitive cells in a culture medium with the imidazolinone herbicide to select for the mutated cells.

13. The method of claim 11 wherein the plant is derived from a seed deposited as ATCC 97535 (93R30B).

## Patentansprüche

1. Ein Zuckerrüben-Pflanzenmaterial, bestehend aus mutierten Zellen mit einem mutierten Acetolactat-Synthase-Gen, kodierend die Synthase, wobei Nukleotide modifiziert sind von Cytosin nach Thymin an Position 562 und Guanin nach Adenin an Position 337, wobei die mutierten Zellen eine Resistenz sowohl gegen Imidazolinon- als auch Sulfonylharnstoff-Herbizide aufweisen, und wobei die Resistenz übertragen wird durch konventionelles Kreuzungs-Züchten von Pflanzen, erzeugt aus den Zellen und die Zellen regenerierbar in eine Pflanze sind.

2. Das Material von Anspruch 1, welches abgeleitet worden ist aus sensitiven Zellen von Eltern-Zuckerrüben-Pflanzen, bezeichnet als CR1-B, hinterlegt als ATCC 97961, welche zugleich eine Resistenz gegen Sulfonylharnstoff-Herbizide und keine Resistenz gegen Imidazolinon-Herbizide aufweisen, durch Kultivierung der sensitiven Zellen in einem Kulturmedium mit dem Imidazolinon-Herbizid, um die mutierten Zellen auszuwählen.

3. Das Pflanzenmaterial gemäß irgend einem der Ansprüche 1 oder 2 als ein Samen- oder Ableger des Samens.

4. Ein Verfahren zum Erzeugen einer Herbizid-Resistenz in einer Zuckerrüben-Pflanze, welches folgendes umfasst:
(a) Exponieren von ersten Zellen an Zuckerrüben-Pflanzen mit einem mutierten Acetolactat-Synthase-Gen kodierend die Synthase, wobei Nukleotide modifiziert sind vom Cytosin nach Thymin an Position 562, gegen ein Imidazolinon-Herbizid in einem Kulturmedium, wobei die ersten Zellen ein homozygotes Merkmal für die Sulfonylharnstoff-Herbizid-Resistenz aufweisen; und
(b) Auswählen nach zweiten Zellen mit einer Resistenz gegen beide Herbizide, wobei die Resistenz übertragen werden kann durch Kreuzungs-Züchtung einer Pflanze enthaltend die zweiten Zellen.

5. Das Verfahren gemäß Anspruch 4, wobei die zweiten Zellen abgeleitet werden aus den ersten Zellen einer Zuckerrüben-Pflanze bezeichnet als 93R30 (SurSur), welche ein homozygoter Nachkomme ist von CR1-B, und letztgenannter hinterlegt ist als ATCC 97961, durch Exponieren der Zellen von 93R30 gegen ein Imidazolinon-Herbizid.

6. Das Verfahren von Anspruch 4, wobei die zweiten Zellen in einer Hinterlegung eines Samens mit der Bezeichnung ATCC 97535 (93R30B) sind.

7. Ein Verfahren zum Verleihen einer Herbizid-Resistenz in einer Zuckerrübe, welches folgendes umfasst:
(a) Exponieren von ersten Zellen an Zuckerrüben-Pflanzen gegen ein Imidazolinon-Herbizid in einem Kulturmedium, wobei die ersten Zellen eine homozygote Sulfonylharnstoff-Herbizid-Resistenz in einem Kulturmedium aufweisen;
(b) Auswählen nach zweiten Zellen mit einer Resistenz für beide Herbizide;
(c) Kultivieren einer ersten Pflanze aus den zweiten Zellen, wobei die Pflanze die Resistenz für die Herbizide mit einem mutierten Acetolactat-Synthase-Gen, kodierend die Synthase, aufweist, wobei Nukleotide modifiziert sind von Cytosin nach Thymin an Position 562 und Guanin nach Adenin an Position 337; und
(d) Kreuzungs-Hybridisieren der ersten Pflanze mit einer zweiten Pflanze, um eine gekreuzte Pflanze zu erzeugen, welche die Resistenz gegen die Herbizide aufweist.

8. Ein Verfahren zum Verleihen von Herbizid-Resistenz auf andere Rüben, welches folgendes umfasst:
(a) Exponieren von ersten Zellen an Zuckerrüben-Pflanzen gegen ein Imidazolinon-Herbizid in einem Kulturmedium, wobei die ersten Zellen eine homozygote Sulfonylharnstoff-Herbizid-Resistenz in einem Kulturmedium aufweisen;
(b) Auswählen nach zweiten Zellen mit einer Resistenz für beide Herbizide;
(c) Kultivieren einer ersten Pflanze aus den zweiten Zellen, wobei die Pflanze die Resistenz für die Herbizide aufweist, mit einem mutierten Acetolactat-Synthase-Gen, kodierend die Synthase, wobei Nukleotide modifiziert sind von Cytosin nach Thymin an Position 562 und Guanin nach Adenin an Position 337; und
(d) Kreuzungs-Züchtung der ersten Pflanze mit einer zweiten Pflanze, um eine gekreuzte Pflanze zu erzeugen, welche die Resistenz gegen die Herbizide aufweist.

9. Das Verfahren von Anspruch 7, wobei die ersten Zellen in Schritt (a) abgeleitet sind von einer Zuckerrübe, bezeichnet als 93R30, welche ein homozygoter Nachkomme ist von CR1-B, wobei letztgenannte hinterlegt wurde als ATCC 97961, durch Exponieren der Zellen von 93R30 gegen ein lmidazolinon-Herbizid.

10. Das Verfahren gemäß Anspruch 7, wobei die zweiten Zellen in einer Hinterlegung eines Samens bezeichnet als ATCC 97535 (93R30B) sind.

11. Ein Verfahren zum Eindämmen von Schädlingen, welche auf Zuckerrüben-Pflanzen wachsen, welches folgendes umfasst.
(a) Pflanzen von Zuckerrüben-Samen in einem Feld, enthaltend Zellen mit einem mutierten Acetolactat-Synthase-Gen, kodierend die Synthase, wobei Nukleotide modifziert sind von Cytosin nach Thymin an Position 562 und Guanin nach Adenin an Position 337, wobei die mutierten Zellen eine Resistenz sowohl gegen Imidazolinon- als auch Sulfonylharnstoff-Herbizide aufweisen, und wobei die Resistenz übertragen wird durch konventionelles Kreuzungs-Hybridisieren von Pflanzen, erzeugt aus den Zellen, um Zuckerrüben-Pflanzen zu erzeugen; und
(b) Verwenden eines Imidazolinon-Herbizids auf der Pflanze in dem Feld, um Schädlinge einzudämmen.

12. Das Verfahren von Anspruch 11, wobei die Pflanze abgeleitet ist aus sensitiven Zellen einer Eltern-Zuckerrüben-Pflanze, bezeichnet als CR1-B, hinterlegt als ATCC 97961, welche zugleich Resistenz gegen Suifonylharnstoff-Herbizide aufweist, und keine Resistenz gegen Imidazolinon-Herbizide, durch Kultivieren der sensitiven Zellen in einem Kulturmedium mit dem Imidazolinon-Herbizid, um nach mutierten Zellen auszuwählen.

13. Das Verfahren von Anspruch 11, wobei die Pflanze abgeleitet ist von einem Samen, hinterlegt als ATCC 97535 (93R30B).

## Revendications

1. Matériel végétal de betterave à sucre consistant en des cellules mutées contenant un gène muté de l'acétolactate synthase codant la synthase dans lequel les nucléotides sont modifiés par le remplacement d'une cytosine par une thymine en position 562 et d'une guanine par une adénine en position 337, dans lequel les cellules mutées sont résistantes aux herbicides à base d'imidazolinone ainsi qu'aux herbicides à base de sulfonylurée et dans lequel la résistance est transmise par un croisement classique des plantes produites à partir des cellules et les cellules peuvent être régénérées en plante.

2. Matériel selon la revendication 1, qui est dérivé de cellules sensibles d'un plant parent de betterave à sucre, désigné par CR1-B, déposé sous le nom ATCC 97961, et étant résistant aux herbicides à base de sulfonylurée sans être résistant aux herbicides à base d'imidazolinone, en cultivant les cellules sensibles dans un milieu de culture avec l'herbicide à base d'imidazolinone pour sélectionner les cellules mutées.

3. Matériel végétal selon l'une quelconque des revendications 1 ou 2, sous la forme d'une semence ou d'une propagule de la semence.

4. Méthode de production d'une résistance aux herbicides dans un plant de betterave à sucre comprenant :
(a) l'exposition de premières cellules de la betterave à sucre contenant un gène muté de l'acétolactate synthase codant la synthase, dans lequel les nucléotides sont modifiés par le remplacement d'une cytosine par une thymine en position 562, à un herbicide à base d'imidazolinone dans un milieu de culture, dans lequel les premières cellules possèdent un caractère homozygote pour la résistance à un herbicide à base de sulfonylurée ; et
(b) la sélection de secondes cellules possédant une résistance aux deux herbicides, dans laquelle la résistance peut être transmise par le croisement d'une plante contenant les secondes cellules.

5. Méthode selon la revendication 4, dans laquelle les secondes cellules dérivent des premières cellules d'un plant de betterave à sucre nommé 93R30 (SurSur), qui est un descendant homozygote de CR1-B, ce dernier étant déposé sous le nom de ATCC 97961, en exposant les cellules de 93R30 à un herbicide à base d'imidazolinone.

6. Méthode selon la revendication 4, dans laquelle les secondes cellules sont dans un dépôt de semence désignée par ATCC 97535 (93R30B).

7. Méthode destinée à conférer une résistance aux herbicides à une betterave à sucre comprenant :
(a) l'exposition de premières cellules d'un plant de betterave à sucre à un herbicide à base d'imidazolinone dans un milieu de culture, dans lequel les premières cellules possèdent une résistance homozygote à un herbicide à base de sulfonylurée dans un milieu de culture ;
(b) la sélection de secondes cellules possédant une résistance aux deux herbicides ;
(c) la croissance d'une première plante à partir des secondes cellules, dans laquelle la plante est résistante aux herbicides avec un gène muté de l'acétolactate synthase codant la synthase, dans lequel les nucléotides sont modifiés par le remplacement d'une cytosine par une thymine en position 562 et d'une guanine par une adénine en position 337 ; et
(d) le croisement de la première plante avec une seconde plante pour produire une plante croisée qui est résistante aux herbicides.

8. Méthode destinée à conférer une résistance à un herbicide à d'autres betteraves à sucre comprenant :
(a) l'exposition de premières cellules d'un plant de betterave à sucre à un herbicide à base d'imidazolinone dans un milieu de culture, dans lequel les premières cellules possèdent une résistance homozygote à un herbicide à base de sulfonylurée dans un milieu de culture ;
(b) la sélection de secondes cellules possédant une résistance aux deux herbicides ;
(c) la croissance d'une première plante à partir des secondes cellules, dans laquelle la plante est résistante aux herbicides avec un gène muté de l'acétolactate synthase codant la synthase, dans lequel les nucléotides sont modifiés par le remplacement d'une cytosine par une thymine en position 562 et d'une guanine par une adénine en position 337 ; et
(d) le croisement de la première plante avec une seconde plante pour produire une plante croisée qui est résistante aux herbicides.

9. Méthode selon la revendication 7, dans laquelle les premières cellules de l'étape (a) dérivent d'une betterave à sucre nommée 93R30, qui est un descendant homozygote de CR1-B, ce dernier étant déposé sous le nom de ATCC 97961, en exposant les cellules de 93R30 à un herbicide à base d'imidazolinone.

10. Méthode selon la revendication 7, dans laquelle les secondes cellules sont dans un dépôt de semence désignée par ATCC 97535 (93R30B).

11. Méthode permettant de contrôler les mauvaises herbes poussant avec des plants de betterave à sucre qui comprend :
(a) la plantation dans un champ de semences de betterave à sucre contenant des cellules contenant un gène muté de l'acétolactate synthase codant la synthase dans lequel les nucléotides sont modifiés par le remplacement d'une cytosine par une thymine en position 562 et d'une guanine par une adénine en position 337, dans lequel les cellules mutées sont résistantes aux herbicides à base d'imidazolinone ainsi qu'aux herbicides à base de sulfonylurée et dans lequel la résistance est transmise par un croisement classique des plantes produites à partir des cellules pour produire un plant de betterave à sucre ; et
(b) l'utilisation d'un herbicide à base d'imidazolinone sur le plant dans le champ pour contrôler les mauvaises herbes.

12. Méthode selon la revendication 11, dans laquelle le plant dérive de cellules sensibles d'un plant parent de betterave à sucre, désigné par CR1-B, déposé sous le nom ATCC 97961, et étant résistant aux herbicides à base de sulfonylurée sans être résistant aux herbicides à base d'imidazolinone, en cultivant les cellules sensibles dans un milieu de culture avec l'herbicide à base d'imidazolinone pour sélectionner les cellules mutées.

13. Méthode selon la revendication 11, dans laquelle le plant dérive d'une semence déposée sous le nom ATCC 97535 (93R30B).
